# EUROPEAN PATENT SPECIFICATION

(11) **EP 0 702 559 B1**
(45) Date of publication and mention of the grant of the patent: **19.03.2003**
(21) Application number: 94920108.1
(22) Date of filing: 07.06.1994
(51) Int. Cl.: A61K 38/02, A61P 21/00, A61P 21/02, A61P 19/02

(54) **MULTIPLE BOTULINUM TOXINS FOR TREATING NEUROMUSCULAR DISORDERS AND CONDITIONS**
Multiple Botulinum Toxine zur Behandlung von neuromuskulären Störungen und Zuständen
TOXINES DE BOTULINUM MULTIPLES UTILISEES DANS LE TRAITEMENT DE TROUBLES ET ETATS NEUROMUSCULAIRES

(30) Priority: 10.06.1993 US 75032
(43) Date of publication of application: 27.03.1996
(73) Proprietor: ALLERGAN, INC., Irvine, California 92623-9534 (US)
(72) Inventor: AOKI, K., Roger, Laguna Hills, CA 92653 (US); GRAYSTON, Michael, W., Irvine, CA 92714 (US); CARLSON, Steven, R., Laguna Niguel, CA 92677 (US); LEON, Judith, M., Laguna Niguel, CA 92677 (US)
(74) Representative: Hutchins, Michael Richard
(86) International application number: US9406418
(87) International publication number: WO94028923

(56) References cited:
- WO-A-93/05800
- WO-A-94/00481
- MICROBIOLOGICAL REVIEW, vol.56, no.1, March 1992, WASHINGTON, DC pages 80 - 99 SCHANTZ ET AL. 'Properties and Use of Botulinum Toxin and Other Microbial Neurotoxins in Medicine'
- JOURNAL OF NEUROLOGY, vol.239, no.1, January 1992, BERLIN pages 16 - 20 HAMBLETON P. 'Clostridium botulinums toxins: a general review ...'
- THE NEW ENGLAND JOURNAL OF MEDICINE, vol.324, no.17, 25 April 1991, BOSTON pages 1186 - 1194 JANKOVIC J. ET AL. 'Therapeutic Uses of Botulinum Toxin' cited in the application

## Description

### FIELD OF THE INVENTION

The present invention provides novel compositions and use for treating diseases of the nervous system, e.g., neuromuscular disorders and conditions, with botulinum toxins. In addition the present invention provides the use of compositions for the treatment of tissue and organ systems which involve the release of neurotransmitters, especially acetylcholine. These cholinergic transmission systems include neuromuscular junctions (muscles), smooth muscles (gut, sphincters, etc.) and secretions (salivation and mucus).

### BACKGROUND OF THE INVENTION

A bacterial toxin, botulinum toxin, in particular botulinum toxin type A, has been used in the treatment of a number of neuromuscular disorders and conditions involving muscular spasm; for example, strabismus, blepharospasm, spasmodic torticollis (cervical dystonia), oromandibular dystonia and spasmodic dysphonia (laryngeal dystonia). The toxin binds rapidly and strongly to presynaptic cholinergic nerve terminals and inhibits the exocytosis of acetylcholine by decreasing the frequency of acetylcholine release. This results in local paralysis and hence relaxation of the muscle afflicted by spasm.

For one example of treating neuromuscular disorders, see U.S. Patent No. 5,053,005 to Borodic, which suggests treating curvature of the juvenile spine, i.e., scoliosis, with an acetylcholine release inhibitor, preferably botulinum toxin A.

For the treatment of strabismus with botulinum toxin type A, see Elston, J.S., et al., *British Journal of Ophthalmology,* 1985, 69, 718-724 and 891-896. For the treatment of blepharospasm with botulinum toxin type A, see Adenis, J.P., et al., *J. Fr. Ophthalmol*., 1990, 13 (5) at pages 259-264. For treating squint, see Elston, J.S., *Eye,* 1990, 4(4):VII. For treating spasmodic and oromandibular dystonia torticollis, see Jankovic et al., *Neurology,* 1987, 37, 616-623.

Spasmodic dysphonia has been treated with botulinum toxin type A. See Blitzer et al., *Ann. Otol. Rhino. Laryngol,* 1985, 94, 591-594. Lingual dystonia was treated with botulinum toxin type A according to Brin et al., *Adv. Neurol*. (1987) 50, 599-608. Finally, Cohen et al., *Neurology* (1987) 37 (Suppl. 1), 123-4, discloses the treatment of writer's cramp with botulinum toxin type A.

The term botulinum toxin is a generic term embracing the family of toxins produced by the anaerobic bacterium *Clostridium botulinum* and, to date, seven immunologically distinct neurotoxins have been identified. These have been given the designations A, B, C, D, E, F and G. For further information concerning the properties of the various botulinum toxins, reference is made to the article by Jankovic and Brin, *The New England Journal of Medicine,* No. 17, 1990, pp. 1186-1194, and to the review by Charles L. Hatheway in Chapter 1 of the book entitled *Botulinum Neurotoxin and Tetanus Toxin,* L. L. Simpson, Ed., published by Academic Press Inc. of San Diego, California, 1989.

The neurotoxic component of botulinum toxin has a molecular weight of about 150 kilodaltons and is thought to comprise a short polypeptide chain of about 50 kD which is considered to be responsible for the toxic properties of the toxin, i.e., by interfering with the exocytosis of acetylcholine, by decreasing the frequency of acetylcholine release, and a larger polypeptide chain of about 100 kD which is believed to be necessary to enable the toxin to bind to the presynaptic membrane. The "short" and "long" chains are linked together by means of a simple disulfid bridge. (It is noted that certain serotypes of botulinum toxin, e.g., type E, may exist in the form of a single chain un-nicked protein, as opposed to a dichain. The single chain form is less active but may be converted to the corresponding dichain by nicking with a protease, e.g., trypsin. Both the single and the dichain are useful in the treatment of the present invention.)

Immunotoxin conjugates of ricin and antibodies, which are characterized as having enhanced cytotoxicity through improving cell surface affinity, are disclosed in European Patent Specification 0 129 434. The inventors note that botulinum may be utilized in place of ricin.

Botulinum toxin is obtained commercially by establishing and growing cultures of *C. botulinum* in a fermenter and then harvesting and purifying the fermented mixture in accordance with known techniques.

Botulinum toxin type A, the toxin type generally utilized in treating neuromuscular conditions, is currently available commercially from several sources; for example, from Port Products Ltd. UK, under the trade name "DYSPORT," and from Allergan, Inc., Irvine, California, under the trade name BOTOX®.

It is one object of the invention to provide novel treatments of neuromuscular disorders and conditions with botulinum toxin type A in combination with botulinum toxin types B, C, D, E, F and G.

### SUMMARY OF THE INVENTION

The present invention provides a composition and its use in the manufacture of a medicament for the treatment of a neuromuscular disorder or condition such as strabismus and other disorders of ocular motility, e.g., comitant and vertical strabismus, lateral rectus palsy, nystagmus, dysthyroid myopathy, etc.; dystonia, e.g., focal dystonias such as spasmodic torticollis, writer's cramp, blepharospasm, oromandibular dystonia and the symptoms thereof, e.g., bruxism, Wilson's disease, tardive dystonia, laryngeal dystonia etc.; other dystonias, e.g., tremor, tics, segmental myoclonus; spasms, such as spasticity due to chronic multiple sclerosis, spasticity resulting in abnormal bladder control, e.g., in patients with spinal cord injury, animus, back spasm, charley horse etc.; tension headaches; levator pelvic syndrome; spina bifida, tardive dyskinesia; Parkinson's and limb (focal) dystonia and stuttering, etc. of a patient, which treatment comprises administering to the patient suffering from said disorder or condition a therapeutically effective amount of botulinum toxin type A in combination with a neurotoxin selected from the group consisting of botulinum toxin types B, C, D, E, F and G. The clinical features of the above-listed neuromuscular disorders and conditions are described in Jankovic and Brin, cited above, and in Quinn, *Disorders of Movement*, Academic Press, 1989.

The present invention further provides compositions of said botulinum toxins in a vehicle suitable for injection of said toxins into the appropriate region of the patient to be treated. Alterations of the vehicle and excipient may include materials designed to retain the injected toxin in the local area.

The present invention further provides a composition and its use in the manufacture of a medicament for the treatment of a neuromuscular disorders or conditions requiring a short duration of therapeutic action (measured in hours or days) or an intermediate duration of therapeutic action (measured in weeks). For example, a short or intermediate duration neurotoxin may be used in procedures to temporarily immobilize a joint or prevent muscle contractions prior to or after surgery or a procedure. Examples of these conditions include: total joint replacement, treatment of compound fractures, joint infections, dislocations. Other uses of a short duration of action product are to aid in joint dislocations, relaxation for physical therapy, alleviation of muscle spasms (to break the cycle of pain and spasm). In addition, a short duration therapy may be useful to determine the muscles involved in curvature of the spine in scoliosis. Unusual spasms of sphincter muscles (ocular, gastrointestinal, vaginal, etc.) may be treated with short duration therapy.

On the other hand, an intermediate duration product may be useful in treating tendon or ligament alignment repair. If a muscle is damaged after trauma, immobilization with an intermediate may help with pain and facilitate healing. In addition, an intermediate duration therapy may be useful in determination of muscles involved in curvature of the spine in scoliosis. Unusual spasms of sphincter muscles (ocular, gastrointestinal, vaginal, etc.) may be treated with intermediate duration therapy.

### DETAILED DESCRIPTION

The botulinum toxins used according to the present invention are botulinum toxins type A, B, C, D, E, F and G.

Each serotype of botulinum toxin has been identified as immunologically different proteins through the sue of specific antibodies. For example, if the antibody (antitoxin) recognizes, that is, neutralizes the biological activity of, for example, type A it will not recognize types B, C, D, E, F or G.

While all of the botulinum toxins appear to be zinc endopeptidases, the mechanism of action of different serotypes, for example, A and E within the neuron appear to be different than that of type B. In addition, the neuronal surface "receptor" for the toxin appears to be different for the serotypes.

The physiologic groups of *Clostridium botulinum* types are listed in Table I.

**Table I.**

| **Physiologic Groups of *Clostridium botulinum*** | | | | | | | |
|---|---|---|---|---|---|---|---|
| **Group** | **Toxin Sero-Type** | **Biochemistry** | **Milk Digest** | **Glucose Fermentation** | **Lipase** | **Phages & Plasmids** | **Phenotypically Related Clostridium (nontoxigenic)** |
| I | A,B,F | proteolytic saccharolytic | + | + | + | + | C. sporogenes |
| II | B,E,F | nonproteolytic saccharolytic psychotrophic | - | + | + | + | |
| III | C,D | nonproteolytic saccharolytic | + | + | + | + | C. novyi |
| IV | G | proteolytic nonsaccharolytic | + | - | - | - | C. subterminale |

These toxin types may be produced by selection from the appropriate physiologic group of *Clostridium botulinum* organisms. The organisms designated as Group I are usually referred to as proteolytic and produce botulinum toxins of types A, B and F. The organisms designated as Group II are saccharolytic and produce botulinum toxins of types B, E and F. The organisms designated as Group III produce only botulinum toxin types C and D and are distinguished from organisms of Groups I and II by the production of significant amounts of propionic acid. Group IV organisms only produce neurotoxin of type G. The production of any and all of the botulinum toxin types A, B, C, D, E, F and G are described in Chapter 1 of *Botulinum Neurotoxin and Tetanus Toxin,* cited above, and/or the references cited therein. Botulinum toxins types B, C, D, E, F and G are also available from various species of clostridia. Currently fourteen species of clostridia are considered pathogenic. Most of the pathogenic strains produce toxins which are responsible for the various pathological signs and symptoms. Organisms which produce botulinum toxins have been isolated from botulism outbreaks in humans (types A, B, E and F) and animals (types C and D). Their identities were described through the use of specific antitoxins (antibodies) developed against the earlier toxins. Type G toxin was found in soil and has low toxigenicity. However, it has been isolated from autopsy specimens, but thus far there has not been adequate evidence that type G botulism has occurred in humans.

In general, four physiologic groups of C. *botulinum* are recognized (I, II, III, IV). The organisms capable of producing a serologically distinct toxin may come from more than one physiological group. For example, Type B and F toxins can be produced by strains from Group I or II. In addition, other strains of clostridial species (C. *baratii,* type F; *C. butyricum*, type E; *C. novyi,* type C₁ or D) have been identified which can produce botulinum neurotoxins.

Preferably, the toxin is administered by means of intramuscular injection directly into a spastic muscle, in the region of the neuromuscular junction, although alternative types of administration (e.g., subcutaneous injection), which can deliver the toxin directly to the affected muscle region, may be employed where appropriate. The toxin can be presented as a sterile pyrogen-free aqueous solution or dispersion and as a sterile powder for reconstitution into a sterile solution or dispersion.

Where desired, tonicity adjusting agents such as sodium chloride, glycerol and various sugars can be added. Stabilizers such as human serum albumin may also be included. The formulation may be preserved by means of a suitable pharmaceutically acceptable preservative such as a paraben, although preferably it is unpreserved.

It is preferred that the toxin is formulated in unit dosage form; for example, it can be provided as a sterile solution in a vial or as a vial or sachet containing a lyophilized powder for reconstituting a suitable vehicle such as water for injection.

In one embodiment, the botulinum toxin is formulated in a solution containing saline and pasteurized human serum albumin, which stabilizes the toxin and minimizes loss through non-specific adsorption. The solution is sterile filtered (0.2 micron filter), filled into individual vials and then vacuum-dried to give a sterile lyophilized powder. In use, the powder can be reconstituted by the addition of sterile unpreserved normal saline (sodium chloride 0.9% for injection).

The dose of toxin administered to the patient will depend upon the severity of the condition; e.g., the number of muscle groups requiring treatment, the age and size of the patient and the potency of the toxin. The potency of the toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

The dosages used in human therapeutic applications are roughly proportional to the mass of muscle being injected. Typically, the dose administered to the patient may be up to about 1,000 units; for example, up to about 500 units, and preferably in the range from about 80 to about 460 units per patient per treatment, although smaller of larger doses may be administered in appropriate circumstances.

As the physicians become more familiar with the use of this product, the dose may be changed. In the botulinum toxin type A, available from Porton, DYSPORT, 1 nanogram (ng) contains 40 U. 1 ng of the botulinum toxin type A, available from Allergan, Inc., i.e., BOTOX®, contains 4 U. The potency of botulinum toxin and its long duration of action mean that doses will tend to be administered on an infrequent basis. Ultimately, however, both the quantity of toxin administered and the frequency of its administration will be at the discretion of the physician responsible for the treatment and will be commensurate with questions of safety and the effects produced by the toxin.

The invention will now be illustrated by reference to the following nonlimiting examples.

In each of the examples, the appropriate muscles of each patient are injected with a sterile solution containing the confirmation of botulinum toxin. Total patient doses range from 80 U to 460 U. Before injecting any muscle group, careful consideration is given to the anatomy of the muscle group, the aim being to inject the area with the highest concentration of neuromuscular junctions , if known. Before injecting the muscle, the position of the needle in the muscle is confirmed by putting the muscle through its range of motion and observing the resultant motion of the needle end. General anaesthesia, local anaesthesia and sedation are used according to the age of the patient, the number of sites to be injected, and the particular needs of the patient. More than one injection and/or sites of injection may be necessary to achieve the desired result. Also, some injections, depending on the muscle to be injected, may require the use of fine, hollow, teflon-coated needles, guided by electromyography.

Following injection, it is noted that there are no systemic or local side effects and none of the patients are found to develop extensive local hypotonicity. The majority of patients show an improvement in function both subjectively and when measured objectively.

### Example 1

### The Use of Botulinum Toxin Type in the Treatment of Tardive Dyskinesia

A patient, suffering from joint dislocation, is treated with a composition having up to 500 units of botulinum toxin type A and a lesser amount of botulinum toxin type B by direct injection of such toxin into the joint. After several hours, the joint is immobilized and muscle contractions are relieved. An increase, or enhancement, of the relief of muscle enhancement caused by the combination of botulinum toxin type A and B for a short duration enables immediate treatment while the long term relief of muscle enhancement enables healing of the reset joint.

### Example 1(a)

The treatment of Example 1 is repeated, except that a combination of botulinum toxin type A and B is used with similar results.

### Example 1(b)

The treatment of Example 1 is repeated, except that a combination of botulinum toxin type A and C is used with similar results.

### Example 1(c)

The treatment of Example 1 is repeated, except that a combination of botulinum toxin type A and D is used with similar results.

### Example 1(d)

The treatment of Example 1 is repeated, except that a combination of botulinum toxin type A and E is used with similar results.

### Example 1(e)

The treatment of Example 1 is repeated, except that a combination of botulinum toxin type A and F is used with similar results.

### Example 1(f)

The treatment of Example 1 is repeated, except that a combination of botulinum toxin type A and G is used with similar results.

### Example 2

### Use of Botulinum Toxin in the Treatment of Spasmodic Torticollis

A patient, suffering from spasmodic torticollis, as manifested by spasmodic or tonic contractions of the neck musculature, producing stereotyped abnormal deviations of the head, the chin being rotated to one side, and the shoulder being elevated toward the side at which the head is rotated, is treated by injection with a composition having up to 300 units, or more, of botulinum toxin type A and up to 300 units, or more, of botulinum toxin type E, in the dystonic neck muscles. After a few hours, the symptoms are substantially alleviated; i.e., the patient is able to hold his head and shoulder in a normal position.

### Example 3

### Use of Botulinum Toxin in the Treatment of Essential Tremor

A patient suffering from essential tremor, which is provoked by maintenance of posture or movement, is treated by injection with therapeutic amounts of botulinum toxin type A and botulinum toxin type B. After two weeks, the symptoms are substantially alleviated.

### Example 3(a)

The treatment of Example 3 is repeated except that a patient suffering from essential tremor is injected with botulinum toxin type A and C. A similar result is obtained.

### Example 3(b)

The treatment of Example 3 is repeated, except that a patient suffering from essential tremor is injected with botulinum toxin type A and D. A similar result is obtained.

### Example 3(c)

The treatment of Example 3 is repeated, except that a patient suffering from essential tremor is injected with botulinum toxin type A and E. A similar result is obtained.

### Example 3(d)

The treatment of Example 3 is repeated, except that a patient suffering from essential tremor is injected with botulinum toxin type A and F. A similar result is obtained.

### Example 3(e)

The treatment of Example 3 is repeated, except that a patient suffering from essential tremor is injected with botulinum toxin type A and G. A similar result is obtained.

### Example 4

### Use of Botulinum Toxin in the Treatment of Spasmodic Dysphonia

A patient, unable to speak clearly due to spasm of the vocal chords, is treated by injection of therapeutic amounts of botulinum toxin type A and therapeutic amounts of botulinum toxin type B. After a few hours, the patient is able to speak clearly.

### Example 4(a)

The treatment of Example 4 is repeated except that a patient suffering from spasmodic dysphonia is injected with botulinum toxin type A and C. A similar result is obtained.

### Example 4(b)

The treatment of Example 4 is repeated, except that a patient suffering from spasmodic dysphonia is injected with botulinum toxin type A and D. A similar result is obtained.

### Example 4(c)

The treatment of Example 4 is repeated, except that a patient suffering from spasmodic dysphonia is injected with botulinum toxin type A and E. A similar result is obtained.

### Example 4(d)

The treatment of Example 4 is repeated, except that a patient suffering from spasmodic dysphonia is injected with botulinum toxin type A and F. A similar result is obtained.

### Example 4(e)

The treatment of Example 4 is repeated, except that a patient suffering from spasmodic dysphonia is injected with botulinum toxin type A and G. A similar result is obtained.

### Example 5

### Use of Botulinum Toxin in the Treatment of Hemifacial Spasm

A patient is suffering from hemifacial spasm as manifested by involuntary rapid synchronous contraction of muscles innervated by the facial nerve on one side. The symptoms are sufficiently advanced to show not only contraction of the muscles around the eye, but twitches spread to involve the other ipsilateral facial muscles. The patient is injected with up to 300 units of botulinum toxin type A and up to 300 units of botulinum toxin type B, and after a few hours, the symptoms are substantially alleviated.

### Example 5(a)

The treatment of Example 5 is repeated except that a patient suffering from hemifacial spasm is injected with botulinum toxin type A and C. A similar result is obtained.

### Example 5(b)

The treatment of Example 5 is repeated, except that a patient suffering from hemifacial spasm is injected with botulinum toxin type A and D. A similar result is obtained.

### Example 5(c)

The treatment of Example 5 is repeated, except that a patient suffering from hemifacial spasm is injected with botulinum toxin type A and E. A similar result is obtained.

### Example 5(d)

The treatment of Example 5 is repeated, except that a patient suffering from hemifacial spasm is injected with botulinum toxin type A and F. A similar result is obtained.

### Example 5(e)

The treatment of Example 5 is repeated, except that a patient suffering from hemifacial spasm is injected with botulinum toxin type A and G. A similar result is obtained.

### Example 6

### Use of Botulinum Toxin in the Treatment of Blepharospasm

A 60-year old woman suffering from idiopathic blepharospasm, a focal form of dystonia involving the orbicularis oculi muscles and producing involuntary eye closure, is treated by injection with a therapeutic amount of botulinum toxin type A and type B into the orbicularis oculi muscle. A total of eight injections, both laterally and medially at the junction of the orbital and preseptal orbicularis is made. Twice as much of the solution is injected laterally as medially. Within twelve to twenty-four hours, detectable muscle weakness begins. Clinical improvement shows in two to three days. The involuntary blinking ceases. The effect of the injections lasts for 75 days.

### Example 6(a)

The treatment of Example 6 is repeated except that a patient suffering from idiopathic blepharospasm is injected with botulinum toxin type A and C. A similar result is obtained.

### Example 6(b)

The treatment of Example 6 is repeated, except that a patient suffering from idiopathic blepharospasm is injected with botulinum toxin type A and D. A similar result is obtained.

### Example 6(c)

The treatment of Example 6 is repeated, except that a patient suffering from idiopathic blepharospasm is injected with botulinum toxin type A and E. A similar result is obtained.

### Example 6(d)

The treatment of Example 6 is repeated, except that a patient suffering from idiopathic blepharospasm is injected with botulinum toxin type A and F. A similar result is obtained.

### Example 6(e)

The treatment of Example 6 is repeated, except that a patient suffering from idiopathic blepharospasm is injected with botulinum toxin type A and G. A similar result is obtained.

Although there has been hereinabove described a use of multiple botulinum toxins for treating neuromuscular disorders and conditions in accordance with the present invention, for the purpose of illustrating the manner in which the invention may be used to advantage, it should be appreciated that the invention is not limited thereto since many obvious modifications can be made, and it is intended to include within this invention any such modifications as will fall within the scope of the appended claims. Accordingly, any and all modifications, variations, or equivalent arrangements which may occur to those skilled in the art, should be considered to be within the scope of the present invention as defined in the appended claims.

## Claims

1. A composition for use in treating a patient suffering from a neuromuscular disorder or condition, said composition comprising a therapeutically effective amount of a combination of at least two neurotoxins selected from a group consisting of botulinum toxin types A, B, C, D, E, F and G.

2. The composition according to claim 1 wherein the selected neurotoxins are botulinum toxin types A and B.

3. The composition according to claim 1 wherein the selected neurotoxins are botulinum toxin types A and C.

4. The composition according to claim 1 wherein the selected neurotoxins are botulinum toxin types A and D.

5. The composition according to claim 1 wherein the selected neurotoxins are botulinum toxin types A and E.

6. The composition according to claim 1 wherein the selected neurotoxins are botulinum toxin types A and F.

7. The composition according to claim 1 wherein the selected neurotoxins are botulinum toxin types A and G.

8. The composition according to any one of the preceding claims wherein the duration of therapeutic activity is suitable for treatment of joint dislocations, relaxation for physical therapy, alleviation of muscle spasm, immobilization of a joint undergoing surgery and for prevention of muscle contractions prior to or after surgery.

9. The composition according to any one of claims 1 to 7 wherein the duration of therapeutic activity is suitable for treating tendon and ligament alignment repair, treatment of scoliosis and spasm of sphincter muscles.

10. The use of at least one neurotoxin selected from botulinum toxin types B, C, D, E, F and G for the manufacture of a medicament for providing a short term enhancement of the therapeutic effect of botulinum toxin type A previously administered to a patient suffering from a neuromuscular disorder or condition.

11. The use according to claim 10 wherein the selected neurotoxin is botulinum toxin type B.

12. The use according to claim 10 wherein the selected neurotoxin is botulinum toxin type C.

13. The use according to claim 10 wherein the selected neurotoxin is botulinum toxin type D.

14. The use according to claim 10 wherein the selected neurotoxin is botulinum toxin type E.

15. The use according to claim 10 wherein the selected neurotoxin is botulinum toxin type F.

16. The use according to claim 10 wherein the selected neurotoxin is botulinum toxin type G.

17. The use of a combination of at least two neurotoxins as defined in any one of claims 1 to 7 for the manufacture of a medicament for the treatment of joint dislocations, relaxation for physical therapy, alleviation of muscle spasm, immobilization of a joint undergoing surgery and for prevention of muscle contractions prior to or after surgery.

18. The use of a combination of at least two neurotoxins as defined in any one of claims 1 to 7 for the manufacture of a medicament for treating tendon and ligament alignment repair, treatment of scoliosis and spasm of sphincter muscles.

## Patentansprüche

1. Verfahren zur Herstellung einer terminal sterilisierten knochenbildenden Vorrichtung zur Implantierung in ein Säugetier, das die folgenden Schritt umfasst:
a) Bereitstellung einer knochenbildenden Vorrichtung in einer luftarmen Umgebung, die in Kombination ein unlösliches Polymerträgermaterial und ein isoliertes, biologisch aktives knochenbildendes Protein umfasst, das zur Induktion einer Ersatzknochenbildung oder Gelenkknorpelbildung in der Lage ist, wenn es einem Säugetier implantiert wird; und
b) Aussetzen der knochenbildenden Vorrichtung aus Schritt (a) einer ionisierenden Strahlung in einer Menge, die zur Sterilisierung der Vorrichtung ausreichend ist, während die biologische Aktivität des Proteins aufrecht erhalten wird, so dass eine terminal sterilisierte knochenbildende Vorrichtung erzeugt wird, die zur Induktion einer Ersatzknochenbildung oder einer Gelenkknorpelbildung in dem Säugetier in der Lage ist.

2. Verwendung einer terminal sterilisierten knochenbildenden Vorrichtung zum Induzieren einer Ersatzknochen- oder Gelenkknorpelbildung an einem vorgewählten Ort in einem Säugetier, wobei die terminal sterilisierte knochenbildende Vorrichtung in Kombination ein unlösliches Polymerträgermaterial und ein isoliertes, biologisch aktives knochenbildendes Protein umfasst, das zum Induzieren einer Ersatzknochenbildung in der Lage ist, wenn es dem Säugetier implantiert wird, wobei die Vorrichtung durch Exposition gegenüber einer ionisierenden Strahlung in einer luftarmen Umgebung sterilisiert wird.

3. Terminal sterilisierte knochenbildende Vorrichtung, die in Kombination ein unlösliches Polymerträgermaterial und ein isoliertes biologisch aktives knochenbildendes Protein umfasst, das zum Induzieren einer Ersatzknochenbildung oder einer Gelenkknorpelbildung in der Lage ist, wenn es einem Säugetier implantiert wird, wobei die Vorrichtung durch Exposition gegenüber einer ionisierenden Strahlung in einer luftarmen Umgebung sterilisiert wird.

4. Verfahren, Verwendung oder Vorrichtung nach einem der vorhergehenden Ansprüche, wobei das Trägermaterial und das knochenbildende Protein in einem Gewichtsverhältnis im Bereich von 1 : 1 bis 250.000 : 1 (wahlweise in einem Bereich von 40 : 1 bis 50.000 : 1) kombiniert werden.

5. Verfahren, Verwendung oder Vorrichtung nach einem der vorhergehenden Ansprüche, wobei das knochenbildende Protein OP-1, OP-2, BMP-2, BMP-3, BMP-4, BMP-5, BMP-6, BMP-8, BMP-9, DPP, Vgl, Vgr-1 oder ein funktionelles Äquivalent hiervon ist.

6. Verfahren, Verwendung oder Vorrichtung nach Anspruch 1 bis 4, wobei das knochenbildende Protein OP-1 oder ein funktionelles Äquivalent hiervon ist.

7. Verfahren, Verwendung oder Vorrichtung nach einem der vorhergehenden Ansprüche, wobei das knochenbildende Protein ein Homodimer ist.

8. Verfahren, Verwendung oder Vorrichtung nach einem der vorhergehenden Ansprüche, wobei das Trägermaterial poröse Teilchen oder zusammengepresste Teilchen umfasst.

9. Verfahren, Verwendung oder Vorrichtung nach einem der vorhergehenden Ansprüche, wobei das Trägermaterial Teilchen mit einer Teilchengröße im Bereich von 70 bis 850 µm (wahlweise im Bereich von 125 bis 450 µm) umfasst.

10. Verfahren, Verwendung oder Vorrichtung nach einem der vorhergehenden Ansprüche, wobei das Trägermaterial Folgendes umfasst:
(a) ein *in vivo* biologisch abbaubares Polymer; oder
(b) ein synthetisches Polymer (das wahlweise Polymilchsäure, Polybuttersäure, Polyglykolsäure oder ein Gemisch hiervon umfasst); oder
(c) ein natürliches Polymermaterial (das wahlweise Hydroxyapatit, Tricalciumphosphat, Collagen oder ein Gemisch hiervon umfasst oder allogener oder xenogener Knochen ist).

11. Verfahren, Verwendung oder Vorrichtung nach Ansprüchen 1 bis 9, wobei das Trägermaterial Collagen umfasst.

12. Verfahren, Verwendung oder Vorrichtung nach einem der vorhergehenden Ansprüche, wobei das Trägermaterial ein bindendes Material (wahlweise Carboxymethylcellulose, Glycerol oder Polyethylenglycol) umfasst.

13. Verfahren, Verwendung oder Vorrichtung nach einem der vorhergehenden Ansprüche, wobei die ionisierende Strahlung Folgendes ist:
(a) Gammastrahlung oder ein Elektronenstrahl; und/oder
(b) in einer Dosis im Bereich von 0,5 bis 4,0 Megarad (wahlweise im Bereich von 2,0 bis 3,5 Megarad) bereitgestellt wird.

14. Verfahren, Verwendung oder Vorrichtung nach einem der vorhergehenden Ansprüche, wobei die terminal sterilisierte knochenbildende Vorrichtung ein Sterilitätssicherungsniveau von ungefähr 10⁻⁶ aufweist.

15. Verfahren, Verwendung oder Vorrichtung nach einem der vorhergehenden Ansprüche, wobei die terminal sterilisierte knochenbildende Vorrichtung zum Induzieren einer Gelenkknorpelbildung an einem avaskulären Ort in der Lage ist.

16. Verfahren, Verwendung oder Vorrichtung nach einem der vorhergehenden Ansprüche, wobei das Säugetier ein Mensch ist.

17. Verfahren, Verwendung oder Vorrichtung nach einem der vorhergehenden Ansprüche, wobei das knochenbildende Protein ein Mitglied der Vg/dpp Unterfamilie der TGF-β Superfamilie ist.

18. Verfahren, Verwendung oder Vorrichtung nach Anspruch 17, wobei das knochenbildende Protein ein konserviertes Sechs- oder Sieben-Cystein-Grundgerüst im C-terminalen Bereich mit anderen Mitgliedern der Vg/dpp Unterfamilie teilt.

## Revendications

1. Une composition à utiliser pour le traitement d'un patient souffrant d'un désordre ou d'un trouble neuromusculaire, cette composition comprenant une quantité thérapeutiquement efficace d'une combinaison d'au moins deux neurotoxines choisies dans un groupe consistant en toxines de type A, B, C, D, E, F et G du botulisme.

2. Composition selon la revendication 1 où les neurotoxines choisies sont les toxines du type A et B du botulisme.

3. Composition selon la revendication 1 où les neurotoxines choisies sont les toxines du type A et C du botulisme.

4. Composition selon la revendication 1 où les neurotoxines choisies sont les toxines du type A et D du botulisme.

5. Composition selon la revendication 1 où les neurotoxines choisies sont les toxines du type A et E du botulisme.

6. Composition selon la revendication 1 où les neurotoxines choisies sont les toxines du type A et F du botulisme.

7. Composition selon la revendication 1 où les neurotoxines choisies sont les toxines du type A et G du botulisme.

8. Composition selon l'une quelconque des revendications précédentes où la durée de l'activité thérapeutique est adéquate pour le traitement des luxations des articulations, la relaxation pour une thérapie physique, un allégement des spasmes musculaires, l'immobilisation d'une articulation subissant une opération chirurgicale et pour l'empêchement de contractions musculaires avant ou après l'opération chirurgicale.

9. Composition selon l'une quelconque des revendications 1 à 7 où la durée de l'activité thérapeutique est adéquate pour le traitement et rétablissement de l'alignement des tendons et des ligaments, le traitement de la scoliose et des spasmes des muscles du sphincter.

10. Utilisation d'au moins une neurotoxine choisie parmi les toxines du type B, C, D, E, F et G du botulisme pour la fabrication d'un médicament pour obtenir une augmentation à court terme de l'effet thérapeutique de la toxine du type A du botulisme au préalable administrée à un patient souffrant de désordre ou de trouble neuromusculaire.

11. Utilisation selon la revendication 10 où la neurotoxine choisie est la toxine botulique du type B.

12. Utilisation selon la revendication 10 où la neurotoxine choisie est la toxine botulique du type C.

13. Utilisation selon la revendication 10 où la neurotoxine choisie est la toxine botulique du type D.

14. Utilisation selon la revendication 10 où la neurotoxine choisie est la toxine botulique du type E.

15. Utilisation selon la revendication 10 où la neurotoxine choisie est la toxine botulique du type F.

16. Utilisation selon la revendication 10 où la neurotoxine choisie est la toxine botulique du type G.

17. Utilisation d'une combinaison d'au moins deux neurotoxines telles que définies dans l'une quelconque des revendications 1 à 7 pour la fabrication d'un médicament pour le traitement de luxations des articulations, la relaxation pour une thérapie physique, l'allégement de spasmes musculaires, l'immobilisation d'une articulation subissant une opération chirurgicale et pour l'empêchement de contractions musculaires avant ou après l'opération chirurgicale.

18. Utilisation d'une combinaison d'au moins deux neurotoxines telles que définies dans l'une quelconque des revendications 1 à 7 pour la fabrication d'un médicament pour le traitement et rétablissement de l'alignement des tendons et des ligaments, le traitement de la scoliose et des spasmes des muscles du sphincter.
